# EUROPEAN PATENT APPLICATION

(11) **EP 3 578 124 A1**
(43) Date of publication of application: **11.12.2019**
(21) Application number: 18176168.5
(22) Date of filing: 05.06.2018
(51) Int. Cl.: A61B 18/14, A61B 18/12, A61B 18/00

(54) **ELECTROPHYSIOLOGY APPARATUS**

(71) Applicant: National University of Ireland Galway, Galway (IE)
(72) Inventor: COFFEY, Kenneth, Galway, H91 KVW6 (IE); O'Brien, Barry, Galway (IE)
(74) Representative: Murgitroyd & Company

(57) **Abstract**

Treating patients with therapeutically effective electroporation requires the use of voltage potentials which when applied to the patient can be painful due to the noxious overstimulation of the afferent pain-receptive nerve fibres. An electrode assembly which includes electrodes for applying effective electroporation voltages, also comprises at least one electrode configured to apply a non-noxious, non-painful electrical stimulation which may be referred to as a "nerve block adjacent to the electroporation site.

## Description

This disclosure relates to apparatus useful in the medical field. The apparatus may be useful for example in the treatment of atrial fibrillation but is not exclusively limited thereto since it may be also useful in other procedures, for example in the treatment of solid tumours.

### Background

Cardiovascular disease is common in the western world and a great deal of research has been carried out in the field to treat and diagnose the various conditions associated with cardiovascular problems. Atrial fibrillation is one heart condition in which the heart beats in an irregular fashion, often much faster than is considered normal. Undiagnosed or untreated atrial fibrillation may lead to weakening of the heart and the potential for heart failure.

Approximately 15 million people in the US and EU have been identified as suffering atrial fibrillation, but current treatments have relatively poor success rates. Drug therapies are usually the first approach to treat the condition, but typically have a success rate of less than 50%. Catheter ablation is then considered, though the success rate of this is also only approximately 50%. These ablation treatments are performed on the inside (endocardial) surface of the heart. In 2016 approximately 250,000 ablations were performed in the US and EU.

Atrial fibrillation can be controlled in suitable subjects by medication or by electrical stimuli (controlled electric shock) to restore a normal rhythm, or by means of catheter ablation of abnormal heart tissue.

According to a known catheter ablation technique, a thin catheter bearing electrodes is introduced to the heart via a blood vessel, and an area of tissue abnormality can be identified according to evaluation of electrical activity of the heart tissue. The area of tissue abnormality can be ablated by introducing high radio frequency energy via the catheter to create localised heat to target the tissue to be ablated. As an alternative to use of heat, a cryoablation technique can be used. Cryoablation may use argon or helium delivered under high pressure via a catheter to achieve temperatures of -55 to -90°C at the catheter tip. Tissue adjacent the tip may be cooled for about 2 minutes leading to subsequent formation of very localised lesions with adjacent tissue being unaffected. Ablation of the abnormal tissue may allow the restoration of normal rhythm of the heart.

It has been assessed that the known catheter ablation techniques may have a success rate of about 50-60%. Therefore, an alternative approach which may improve on that rate and be suitable for patients with either paroxysmal or persistent atrial fibrillation would be useful. It has also been recognised that the aforesaid radiofrequency or cryoablation techniques may introduce thermal damage to the myocardium which damage offsets benefits of the technique and impairs outcomes for the patients.

Currently catheter ablation for atrial fibrillation is performed using either radiofrequency (RF) or cryo energy sources. These ablations are typically performed on the inside surface of the heart (endocardial) with the intention of locally destroying the pathways of the stray electrical signals that cause fibrillation, mostly in the ostia of the pulmonary veins in the left atrium. However, these techniques cause significant collateral damage to healthy tissue which can result in impaired cardiac function and can induce other arrhythmias such as atrial tachycardia. In addition, as mentioned earlier, one year success rates are only approximately 50%. While there have been some previous efforts to ablate the ganglionated plexi using an epicardial approach, these have had limited success also due to the collateral damage caused by the RF energy used. In addition, the transverse sinus ganglionated plexi are not readily ablated due to the risk of RF or cryo damage to the coronary arteries, aorta and vena cava. The technology proposed in this disclosure differs from these approaches in that it is focused at the ganglionated plexi located on the epicardial surface root cause of the fibrillation signals than existing endocardial methods. In addition it is aiming to avoid the creation of the transmural RF lesions that contribute to atrial tachycardia and flutter and that can damage arteries.

The current energy sources used for ablation (radiofrequency and cryo energy) present a hazard to the arteries and veins, including the pulmonary artery, the aorta, the right and left coronary arteries and the superior vena cava. Damage due to RF or cryo energy can cause lesions leading to thrombus, restenosis, and occlusion as well as potentially causing perforation.

The following publications may be useful in understanding the background to this disclosure.
***1.*** BJ Scherlag et al. Autonomically Induced Conversion of Pulmonary Vein Focal Firing Into Atrial Fibrillation. J Am Coll Cardial 2005; 45: 1878-1876*.*
**2.** C Pappone et al. Pulmonary Vein Denervation Enhances Long- Term Benefit After Circumferential Ablation for Paroxysmal Atrial Fibrillation. Circulation 2004: 109: 327-334*.*
**3.** J Armour et al. Gross and Microscopic Anatomy of the Human Intrinsic Cardiac Nervous System. The Anatomical Record 1997; 247: 289-298*.*
**4.** B Cui et al. Acute effects of ganglionated plexi ablation on sinoatrial nodal and atrioventricular nodal functions. Autonomic Neuroscience. Basic and Clinical 2011, 161: 87-94*.*

It is being increasingly recognized that sites on the outside (epicardial) surface of the heart are contributing to initiation and maintenance of atrial fibrillation^{1.2}. These specific sites are where clusters of autonomic nerve cells interface with each other and with the heart itself. These clusters are known as ganglions and are typically embedded within fat pads on the epicardial surface of the heart. These ganglia are essentially junctions between autonomic nerves originating from the central nervous system and autonomic nerves innervating their target organs, such as the heart. Each ganglion contains multiple neurons, typically ranging from 10 to 200³.

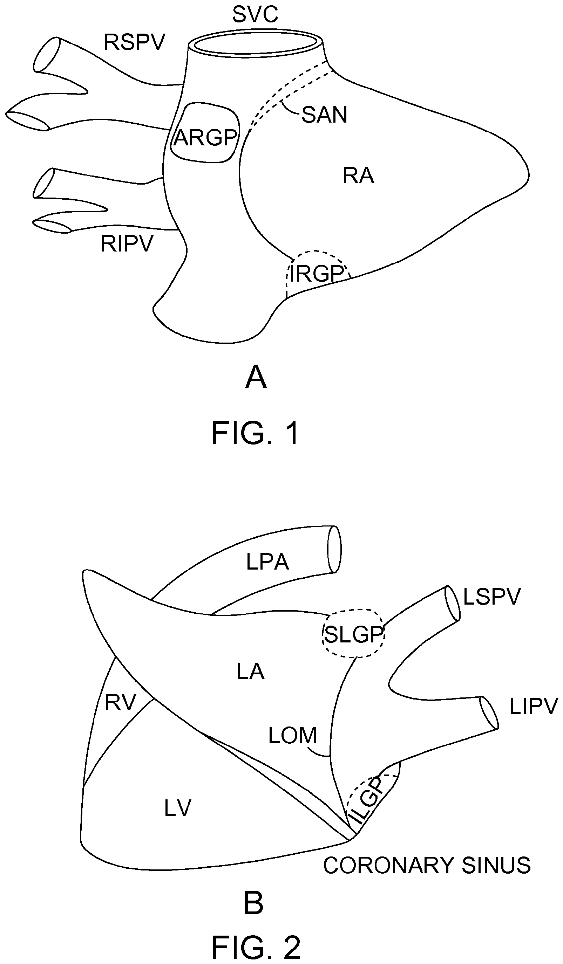

The cardiac ganglia exist throughout the epicardial fat within the pericardial space. However groups of several cardiac ganglia form plexi that interconnect and coalesce in specific locations (known as ganglionated plexi). From an electrophysiology perspective the location of these ganglionated plexi is often defined relative to the pulmonary veins (Figs. 1 and 2), i.e. the four common retroatrial ganglionated plexi ⁴ being:
the **superior left ganglionated plexi** (SLGP) being located on top of the left atrium near the junction of the left atrium and the left superior pulmonary vein;
the **anterior right ganglionated plexi** (ARGP) being located just anterior and inferior to the right superior pulmonary vein in proximity to the junction of the right superior pulmonary vein and both atria;
the **inferior left ganglionated plexi** (ILGP) being located at the inferior aspect of the posterior wall of the left atrium, at the junction of the left inferior pulmonary vein and left atrium;
the **inferior right ganglionated plexi** (IRGP) being located at the inferior aspect of the posterior wall of the left atrium, near the junction of the inferior vena cava and both atria. There are additional ganglia located in fat pads in and around the transverse sinus.

Electroporation may be considered as an alternative to ablation. This is an athermal process that involves application of high voltage direct current pulses for very short durations which avoids heat build-up, for example applying 1000 volts for 100 µs. However, the efficacy of electroporation is also temperature dependent, with the threshold for electroporation being decreased at elevated temperatures. Electroporation could selectively ablate neurons in a targeted manner without discernible collateral damage to surrounding structures such as myocardium and arterial or venous tissue.

Electroporation is used to transform cells either *in vitro* or *in vivo.* Electroporation is the application of high voltage or current pulses across tissue or cells; the energy creates "pores" in the cell walls. If the cells recover, the mechanism is reversible. This reversible mechanism is important for drug delivery and gene delivery applications. If the cells don't recover, this is known as irreversible. Irreversible electroporation is an important treatment option in that ablation is performed without creating potentially adverse thermal effects. More recently, our unpublished pre-clinical data shows electroporation is a promising treatment for Atrial Fibrillation {AF) in that certain cells and tissue structures can undergo Irreversible electroporation (such as neuronal cells) while cells that are to be preserved (such as myocardial cells) experience reversible electroporation and are not permanently damaged/ablated.

One significant downside to treating patients with electroporation is that the voltage potentials applied to the patient can be painful due to the noxious overstimulation of the afferent pain-receptive nerve fibres. Therefore the anticipated painful sensation experienced under the voltage levels of electroporation to reach therapeutic levels requires the patient to be placed under general anaesthesia for treatment with the well-known associated risks such as anaphylaxis..

### Summary

This disclosure provides electrophysiology apparatus configured to decrease or mitigate the pain experienced by the patient during the electroporation treatment by applying a non-noxious, non-painful electrical stimulation. This stimulation may be referred to as a "nerve block". The apparatus and procedures disclosed herein may enable the use of methods avoiding traditional general anaesthesia, by enabling or facilitating conscious sedation, thereby reducing risks, recovery time and providing lower overall treatment costs. This disclosure extends to all procedures in which a patient may obtain pain relief by provision of a concomitant "nerve block" during electroporation treatment of tissues. In particular the present disclosure provides an electrode assembly for electroporation of tissue with concomitant nerve block when in use with an electrophysiology apparatus comprising a high voltage pulsed DC supply. The electrode assembly may comprise multiple electrodes which can be selectively connected to the high voltage pulsed DC supply to effect electroporation or nerve blocking stimuli.

The electrode assembly is controlled by a control system configured to regulate the applied voltage and deliver electrical pulses which control system is connected with selected electrodes such that certain ones of the electrodes provide nerve blocking stimuli and other selected electrodes effect electroporation.

Thus according to a first aspect an electrophysiology apparatus comprises a high voltage pulsed DC supply, and an electrode assembly connectable with the high voltage pulsed DC supply to provide an electroporation electrode, wherein the electrode assembly comprises (i) at least one electroporation electrode configured to carry a current density sufficient to effect electroporation of tissue in a locus around the at least one electroporation electrode, and (ii) at least one other electrode configured to carry a current density that is insufficient to effect electroporation of tissue, wherein the at least one electroporation electrode and the at least one other electrode are configured to operate concomitantly and the at least one other electrode is configured to deliver nerve blocking stimulation by way of electrical pulses in the range 4000 Hz to 10,000 Hz in the vicinity of the tissue in the locus around the at least one electroporation electrode.

The electrode assembly may be presented for use using a catheter. For example the electrodes which together form the electrode assembly may be positioned upon the exterior surface of the catheter. Alternatively some or all of the electrodes may be positioned inside the catheter.

The electrode assembly may comprise at least one high current density electrode and a corresponding dispersive electrode (sometimes called an "indifferent electrode" often in the form of a patient back pad) to be used in conjunction with the at least one high current density electrode, when connected to a pulsed direct current electrical supply for electroporation of tissue.

The configuration of the electrode assembly may be of various forms having regard to the intended target locus for tissue treatment and typically will comprise multiple electrodes appropriately connected via conductive means and selectively operable to deliver either electroporation pulses or pain modulation electrical pulses (nerve blocking stimulation in the range 4000 Hz to 10,000 Hz).

The electrode assembly may be configured as a tubular tip attachable to a catheter and bearing a plurality of electroporation electrodes configured to carry a current density sufficient to effect electroporation of tissue, thereby forming a "finger"-shaped electrode.

Alternatively the electrodes may be mounted on modular tubular frame parts which may be assembled into a framework supporting an array of electrodes.

The disclosed methods and apparatus will now be further described with reference to the accompanying drawings in which:
Fig. 1 illustrates a schematic view of a heart showing location of retroarterial ganglionated plexi (anterior right and inferior right ganglionated plexi);
Fig. 2 illustrates a schematic view of a heart showing location of retroarterial ganglionated plexi (superior left and inferior left ganglionated plexi);
Fig. 3 illustrates schematically a supine patient receiving monopolar electroporation treatment using an active high current density electrode and a corresponding dispersive electrode in the form of a patient back pad;
Fig. 4 illustrates schematically a supine patient receiving bipolar electroporation treatment using two corresponding active high current density electrodes closely positioned at the tissue treatment site;
Fig. 5 illustrates schematically a monopolar electroporation treatment with concomitant nerve blocking;
Fig. 6 illustrates schematically a bi-polar electroporation treatment with concomitant nerve blocking;
Fig. 7 illustrates a catheter tip with an electrode assembly comprising four transmit (Tx) electrodes for use in electroporation (not in accordance with the invention);
Fig. 8 illustrates a catheter tip with an electrode assembly comprising four transmit (Tx) electrodes for use in electroporation and two electrodes for use in concomitant nerve blocking during electroporation;
Fig. 9 illustrates an electrode assembly of the so-called "glove" configuration comprising multiple (16 in this embodiment) transmit (Tx) electrodes for use in epicardial electroporation with selected ones of the electrodes being used to deliver nerve blocking;
Fig. 10 illustrates an electrode assembly of the so-called "glove" configuration comprising multiple (16 in this embodiment) transmit (Tx) electrodes for use in epicardial electroporation with additional electrodes being used to deliver nerve blocking; and
Fig. 11 illustrated schematically apparatus for use with an electrode assembly to perform a procedure including electroporation treatment with concomitant nerve blocking.

Embodiments of different methods of electroporation of tissue will now be described as illustrative examples. While this is specifically described in relation to electroporation of heart tissue for stimulation for atrial fibrillation treatment, this technique could equally apply to any accessible target tissue structures in the body.

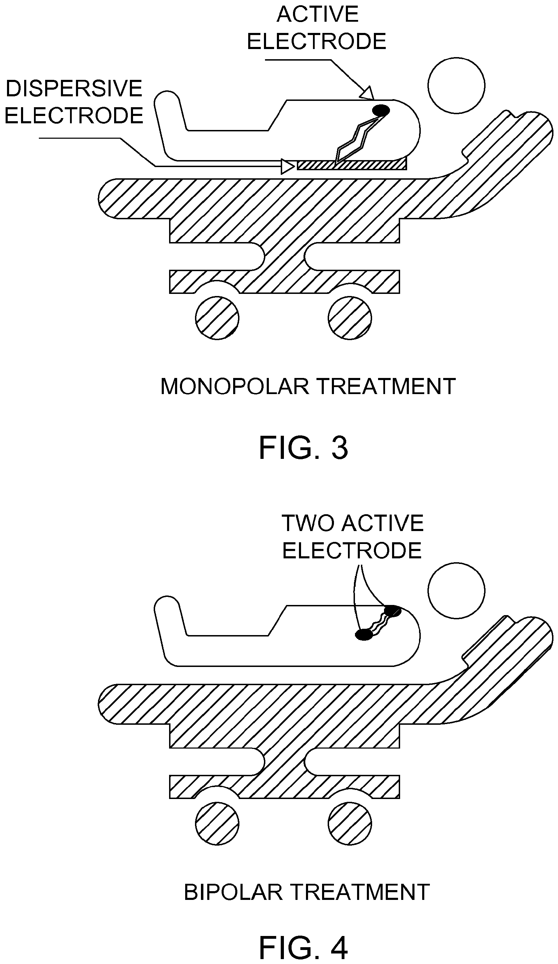

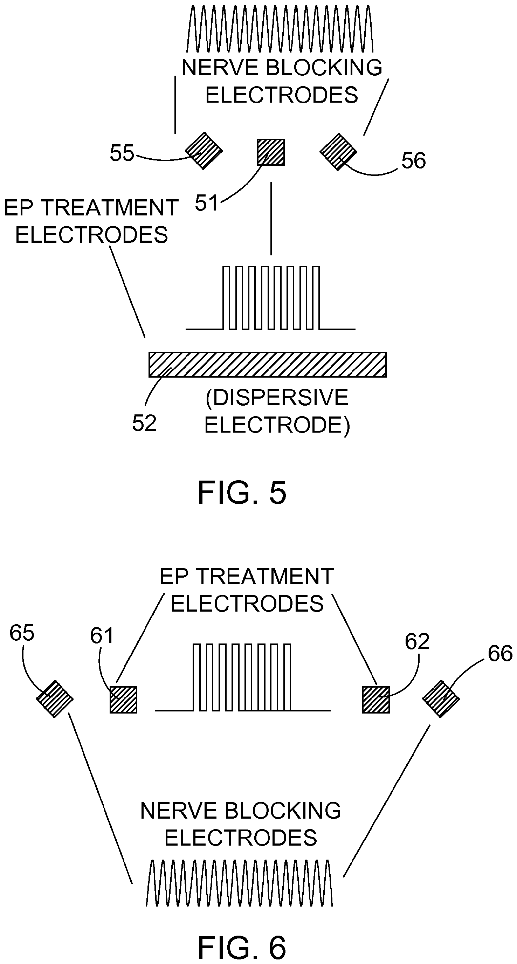

In one embodiment of the electrophysiology apparatus for electroporation with concomitant "nerve block" (Figs. 3 and 5) the electrode assembly is configured for use in a procedure referred to here as a "monopolar" technique, the characteristic features of the electrode assembly thereof for electroporation treatment including the following:
the embodiment comprises two corresponding electrodes of which
- one electrode is significantly larger than the other.
- the smaller electrode will, therefore, carry a much higher current density.
- the higher current density at the smaller electrode will act on the tissue for electroporation treatment.
- the lower density at the larger electrode (dispersive electrode) will make the energy levels such that there is no therapeutic effect. The patient may experience a sensation at the dispersive electrode, but due to its size and lower current density, it should not be painful or noxious.

In a second embodiment of the electrophysiology apparatus for electroporation with concomitant "nerve block" the electrode assembly is configured for use in a procedure referred to here as use a "bi-polar" technique where the electrical energy flows between two electrodes for a localized approach. These electrodes are often the same size.

This technique has the following characteristics.
- The tissues treated are localized.
- The electrodes are often equal, or near equal size.

In the "bi-polar" technique two electrodes may be positioned at the tissue treatment site near, or adjacent to each other, and operated to effect electroporation of tissue.

This invention aims to modulate pain during the electroporation procedure to perhaps have the patient undergo only sedation.

Melzack R and Wall PD proposed the "Gate" theory of Pain in 1965 that is still (with some limitations) accepted as the way the brain and body experience pain, and ways of blocking, or modulating pain. (Science. 1965 Nov 19;150 (3699):971-9. "Pain mechanisms: a new theory." Melzack R, Wall PD. Their theory suggested that pain is experienced in the brain and that the spinal cord carries signals to the brain, but the spinal cord is limited in respect of the numbers of signals that can be carried. It can almost be thought of as a 'limited bandwidth" problem. The Gate Theory has been studied in detail, perhaps modified slightly, but continues to stand the test of time. ("The golden anniversary of Melzack and Wall's gate control theory of pain: Celebrating 50 years of pain research and management", Katz J, Rosenbloom, Pain Res Manag. 2015 Nov-Dec;20(6):285-6).

In addition, specific sine wave electrical stimulation for a "reversible nerve block" has been studied in detail to temporarily and reversibly block the action potential for peripheral nerves. The mechanism is either a depolarization, or "hyperpolarization" of the nerve fibres. The sinusoidal electrical stimulation at 4000Hz to 10,000Hz is thought to be ideal for this mechanism. Nearing and above 10,000Hz, localized tissue heating becomes an issue resulting in pain, or even tissue damage.

In an embodiment, referring to Fig. 5 which schematically illustrates a mono-polar electroporation with concomitant nerve block stimulation, a first pair of cooperating electrodes **51**, **52** of differing sizes, comprising a smaller electrode 51 providing a higher current density and a larger (dispersive) electrode 52 providing a lower current density are arranged at a selected spacing, where the dispersive electrode 52 may be provided as a patient back pad, and the smaller electrode 51 may be provided upon a catheter, and at the periphery of the projected treatment locus around the smaller electrode 51, another pair of corresponding electrodes **55**, **56** are positioned to deliver concomitant nerve blocking stimulation.

The greatest amount of pain will be experienced at the area treated by the smaller electroporation treatment electrode. Therefore, the nerve blocking electrical stimulation (4000Hz-10,000Hz) will be delivered adjacent to, or surrounding the treatment area where the smaller electroporation electrode 51 is positioned.

In an embodiment, referring to Fig. 6 schematically illustrating bi-polar electroporation with concomitant nerve block stimulation, a first pair of corresponding electrodes **61**, **62** are arranged at a spacing suitable for electroporation of tissue (not shown), and a further pair of corresponding electrodes **65**, **66** are juxtaposed to the first pair, at the periphery of the spacing between the first pair of electrodes for the purpose of nerve blocking. A controllable electrical supply (not shown) is appropriately connected respectively to the electrode pairs 61, 62 and 65, 66 via conductive means and selectively operable to deliver either electroporation pulses or pain modulation (nerve blocking stimulation) electrical pulses in the range 4000 Hz to 10,000 Hz. In other embodiments multiple pairs of electroporation electrodes and nerve blocking electrodes may be provided in an electrode assembly to enable concomitant electroporation with nerve blocking on an area of tissue.

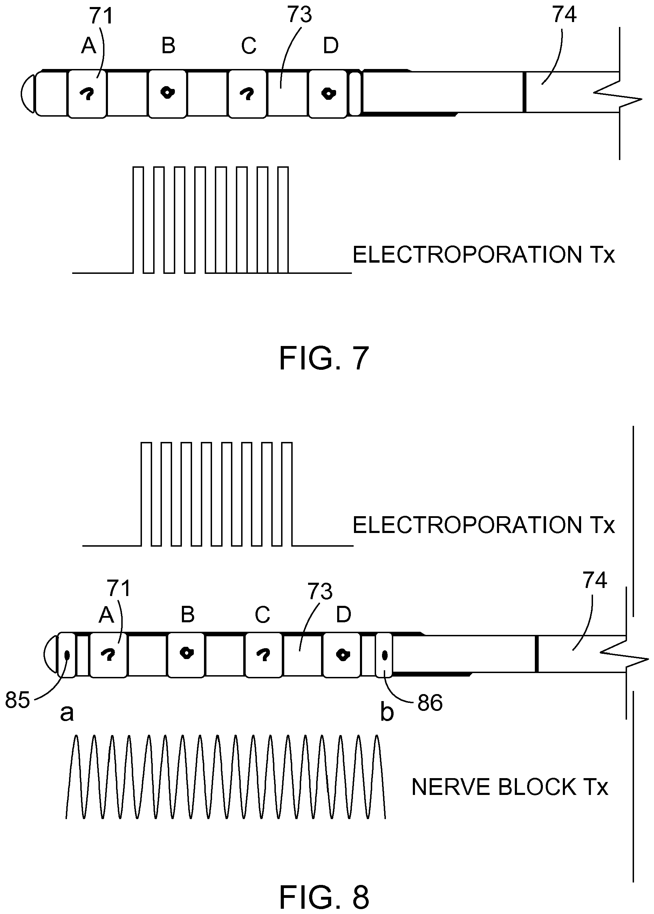

Referring to Fig. 7, a catheter tip with an electrode assembly (finger electrode) comprising four transmit (Tx) electrodes **71** labelled A, B, C D for use in electroporation is shown without cooperating electrodes for nerve blocking. Such a "finger electrode" may be used to treat the fat pads on the epicardial surface of the heart to selectively ablate the ganglionated plexi while preserving the Myocardium. The treatment may be for Atrial Fibrillation.

The electrodes "A", "B". "C", and "D" can be independently active, or connected to one or more, or to all other electrodes. In a bi-polar arrangement, the current could flow from "A" to "B", "A" to "C", or "A" to "D". There could be any permutation of the four electrodes.

Alternatively, all electrodes could be joined together inside the catheter, or the electroporation device itself with an inactive "dispersive" electrode.

Referring now to Fig.8 an embodiment of the invention is shown where the finger electrode of Fig. 7 is modified with additional outer electrodes **85**, **86** which provide the nerve blocking stimulation.

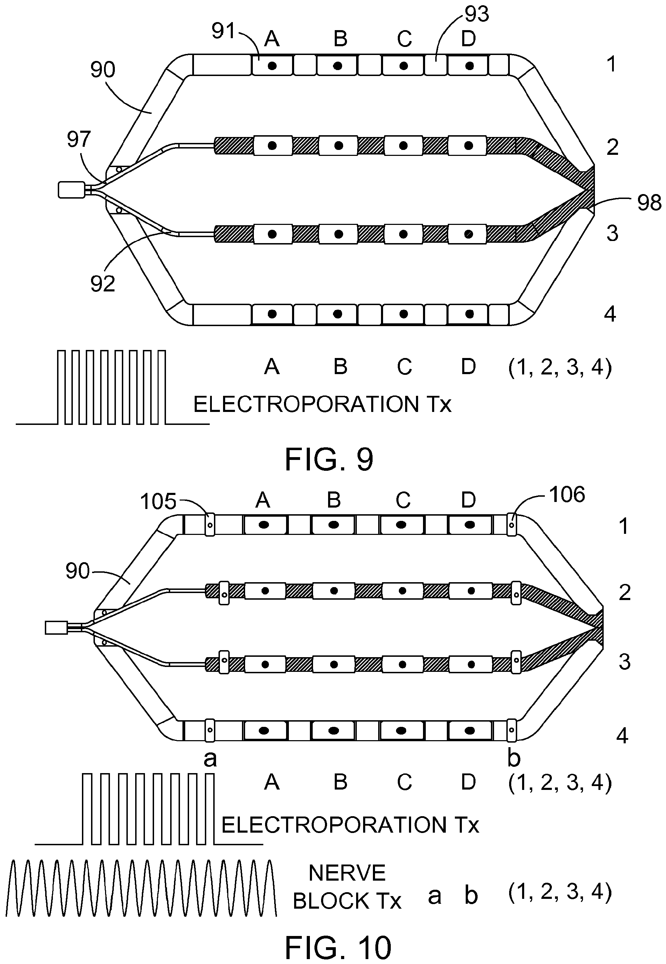

Another form of electrode assembly is illustrated in Fig. 9, in a so-called "glove" configuration and comprising a modular polygonal frame **90** formed from tubular parts wherein a plurality of tubular parts are provided with a series of Tx electrodes **91**, for example as labelled A, B, C, D, and the tubular parts are fixed together into a polygonal frame with two parallel sides **93**, **94.** Additional parallel tubular "bridging" elements **92** are attached at opposite apexes **97**, **98** to provide a 4 X 4 array of electrodes A, B, C, D and 1 through 4.

The electrode assembly of Fig. 9 forms the basis of the embodiment shown in Fig, 10, where additional nerve blocking electrodes **105** and **106** are added at each end of each of the series of electroporation electrodes 91 (A, B, C D) so that any target area subject to electroporation by the "glove electrode" array is concomitantly stimulated to provide nerve block and thereby inhibition of perception of pain.

It should be noted that the use of "monopolar and bipolar" is often used in the literature for describing the polarity of a waveform. This is not the case in this disclosure.

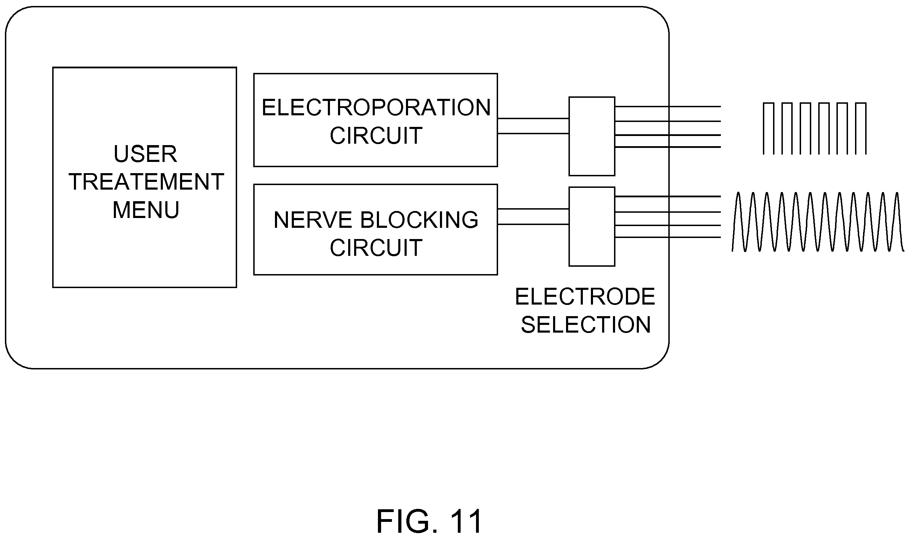

Referring to Figure 11, electrophysiology apparatus for use with an electrode assembly as disclosed herein to perform a procedure including electroporation of tissue with concomitant nerve blocking includes an electroporation circuit and a nerve blocking circuit each connected to respective outputs of a generator such that an output of the generator delivers a high voltage (for example about 1000V square DC pulses) to selected electroporation electrodes through switching circuitry, and another output of the generator selectively delivers a lower voltage (for example about 50V sine wave outputs (for example about 4,000 Hz) by means of switching circuitry to nerve blocking electrodes.

A User Treatment menu allows a user to make a selection of various parameters and treatments.

The timing of delivered voltage is controlled internally through a microprocessor and software/firmware that enables the nerve stimulation to occur prior to the electroporation treatment and continue to complete at the same time, or the duration to be longer than the electroporation treatment.

All embodiments of the electrophysiology apparatus disclosed herein that is configured to effect electroporation of tissue comprises a high voltage pulsed DC supply, and may use at least one catheter including a catheter tip and electrode assembly connectable with the high voltage pulsed DC supply.

A suitable catheter may have an internal lumen (throughbore) for passage of electrical conductors or fluids.

The apparatus can be configured such that in use all of the electrodes on a single catheter tip have the same polarity and may be operated simultaneously. The apparatus may also be configured such that individual electrodes can be operated independently in order that voltage levels and polarity can be selectively controlled.

Electrodes may be designed to minimise sharp points or edges and preferably have smooth contours so that changes in surfaces, edges, ends or corners are rounded in order to reduce the risk of point concentration of the electric field.

An electrode for mounting upon a catheter tip may be of tubular form, and may be a short hollow cylinder having an outside diameter which may be from 2 to 4 mm but can lie in the range of from 1 mm up to 8 mm, and having a length of from 2 to 10 mm but can range from 0.5 mm up to 20 mm.

An electrode for mounting upon a catheter tip may be of tubular form and have one or more irrigation holes in the cylindrical sidewall allowing transmission of fluid supplied via the catheter to the external surface of the electrode. Such irrigation holes may be used to direct conductive saline towards target tissue, thereby increasing the distance over which the electric field will develop. Unlike conventional electrodes, such irrigation is not required for cooling of the electrode. Thus in alternative embodiments the irrigation hole(s), could be positioned in the surface of the catheter adjacent to the electrode, rather than being within the sidewall of the electrode.

Electrodes may be partially insulated to preferentially direct the electric field in a desired direction, and reduce the effect of the electric field in other directions.

In embodiments, an expandable balloon may be use to provide the desired electrical insulation when appropriately positioned to cover parts of the electrode, leaving selected surface(s) exposed. Use of a balloon may also serve to move the electrode(s) closer to a tissue surface to be treated.

When a catheter tip is used to mount the electrode assembly the apparatus may comprise a sheath for electrical field blocking. The sheath is designed to prevent electrical fields from being absorbed by collateral structures near to the target tissue. The sheath may be configured to fit over the catheter tip and electrode assembly and may be fenestrated so as to have a plurality of openings for selective electric field emission. The openings serve as "windows" which may expose one or more electrodes to allow selective directional control for the electrical field emissions. The sheath may be adjusted circumferentially or longitudinally to influence electrical field emission.

The sheath may be fenestrated by provision of multiple holes or apertures positioned along the length and around the circumference of the sheath. It will be understood that rotational or axial movement of the sheath can provide for directing the field to different locations.

The sheath may comprise a metal reinforced polymer, such as a metal-braided polymer tubes of a type already widely used in the field for catheter structures.

US 6 635 047 B2, as an example, discloses a metal braid reinforced polymer tube for use as a coronary guide catheter. The parts of the tube containing the metal braid structure acts as a Faraday cage such that an electrical field does not pass through it.

The disclosed apparatus is useful for physiological modification of tissue, and can be operated by introduction of the electrode assembly to a surgical site, and control of an electrical power source to deliver powerful direct current pulses of short duration whereby a high voltage is applicable to tissue without the drawback of heat associated with electric current. This "electroporation" procedure modifies tissue such that the tissue is initially rendered more permeable and at increased energy levels (higher voltages and longer pulse durations) the electroporation can be controlled to cause cell death and thereby achieve an effect equivalent to tissue ablation.

Electroporation of target tissue is achievable using the apparatus disclosed herein, in that when electrodes of opposite charge are brought into contact with tissue a potential difference is applied across the tissue, and that electrode contact completes a circuit, which circuit includes the high voltage pulsed DC supply, electrodes and the tissue, and thereby accomplishes the electroporation of target tissue with no significant or enduring damage to tissue adjacent to the target tissue

Nerve blocking at about the same time, or otherwise synchronized with electroporation pulses during electroporation, is achievable using the apparatus disclosed herein, in that a high frequency (4000 Hz to 10,000 Hz) connected with selected electrodes such that certain ones of the electrodes provide nerve blocking stimuli. Nerve blocking may be timed by use of a controller such as a microprocessor, and software/firmware such that the nerve stimulation is initiated prior to the electroporation treatment, continues during the electroporation treatment, and either terminates at the same time as, or later than completion of the electroporation treatment.

Ganglionated plexi can be targeted by a localised electrical field generated around and between catheter tip and electrode assemblies as disclosed herein. Ganglionated plexi contain neurons which may be preferentially susceptible to electroporation in comparison to the myocardium. This susceptibility may be attributable to relative size difference between the two cell types; cardiac myocytes are typically 10 -20 µm in diameter, and 50-100 µm in length whilst a neuron soma (body) can be from 4 to 100 µm in diameter, with the axons extending over 1000 µm in length. Control of the electrical field strength and extending the duration of pulses can shift the electroporation mechanism from reversible to irreversible electroporation. Taking account of the preferential susceptibility of neurons, appropriate control of electrical field strength and the duration of pulses applied to such differing cell types is found to result in selective irreversible electroporation of ganglionated plexi, with the adjacent cardiac myocytes being primarily reversibly electroporated.

It will be appreciated that the embodiments disclosed here indicative designs for the purposes of illustration and that other designs with different overall shape and electrode arrangements are possible, so that these examples are non-limiting and attention is directed to the scope of the claims hereinafter appearing.

## Claims

1. An electrode assembly (90) for use in an electrophysiology apparatus comprising a high voltage pulsed DC supply, wherein the electrode assembly (90) is connectable with the high voltage pulsed DC supply to provide an electroporation electrode, and wherein the electrode assembly comprises (i) at least one electroporation electrode (91) configured to carry a current density sufficient to effect electroporation of tissue, and (ii) at least one other electrode (105, 106) configured to carry a current density that is insufficient to effect electroporation of tissue, ***characterised in that** the* at least one electroporation electrode (91) and the at least one other electrode (105, 106) are configured to operate concomitantly and the at least one other electrode (105, 106) is configured to deliver nerve blocking stimulation by way of electrical pulses in the range 4000 Hz to 10,000 Hz.

2. An electrode assembly for use in an electrophysiology apparatus comprising a high voltage pulsed DC supply, wherein the electrode assembly is connectable with the high voltage pulsed DC supply to provide an electroporation electrode, and the electrode assembly is configured as a tubular tip (73) attachable to a catheter (74) and bearing a plurality of electroporation electrodes (71) configured to carry a current density sufficient to effect electroporation of tissue, and further electrodes (85, 86) configured to deliver nerve blocking stimulation by way of electrical pulses in the range 4000 Hz to 10,000 Hz.

3. An electrode assembly as claimed in claim 1 or claim 2, comprising at least one high current density electrode (51) and a corresponding dispersive electrode (52) to be used in conjunction with the at least one high current density electrode, when connected to a direct current electrical supply for electroporation of tissue.

4. An electrode assembly as claimed in claim 1 or claim 2, comprising at least a first pair of spaced apart corresponding electrodes (61, 62) for electroporation of tissue, and a further pair of corresponding electrodes (65, 66) juxtaposed to the first pair of spaced apart corresponding electrodes (61, 62), at the periphery of the spacing between the first pair of electrodes to deliver nerve blocking stimulation by way of electrical pulses in the range 4000 Hz to 10,000 Hz.

5. An electrode assembly as claimed in claim 1, comprising a modular polygonal frame (90) wherein a plurality of tubular parts are provided with a series of Tx electroporation electrodes (91), and the tubular parts are fixed together to form a polygonal frame with two parallel sides (93, 94), and parallel tubular "bridging" elements (92) are attached at opposite apexes (97, 98) to provide a 4 X 4 array of Tx electrodes ***characterised in that*** additional nerve blocking electrodes (105,106) are located at each end of each of the series of electroporation electrodes (91).

6. An. electrode assembly according to any one of claims 1-5, wherein the high voltage of the pulsed DC supply is about 1000 V.
